(19) **Europäisches Patentamt / European Patent Office / Office européen des brevets**

(11) **EP 1 271 133 B1**

(12) **EUROPEAN PATENT SPECIFICATION**

(45) Date of publication and mention
of the grant of the patent:
**28.05.2008 Bulletin 2008/22**

(51) Int Cl.:
$G01N\ 21/78$ (2006.01)    $G01N\ 33/50$ (2006.01)
$C09K\ 11/07$ (2006.01)

(21) Application number: **01908141.3**

(22) Date of filing: **28.02.2001**

(86) International application number:
**PCT/JP2001/001502**

(87) International publication number:
**WO 2001/063265 (30.08.2001 Gazette 2001/35)**

(54) **Method of measurement based on fluorescence energy transfer using a long-lived fluorescence donor**

Messverfahren basierend auf Fluoreszenz- Energie-Transfer mit einem Donor langer Fluoreszenzlebenszeit

Méthode de mesure basée sur la fluorescence par transfert d'énergie utilisant un donneur fluorescent à longue durée de vie

(84) Designated Contracting States:
**AT BE CH CY DE DK ES FI FR GB GR IE IT LI LU MC NL PT SE TR**

(30) Priority: **28.02.2000 JP 2000050868**

(43) Date of publication of application:
**02.01.2003 Bulletin 2003/01**

(73) Proprietors:
• **DAIICHI PURE CHEMICALS CO., LTD.**
**Chuo-ku**
**Tokyo 103-0027 (JP)**
• **Nagano, Tetsuo**
**Suginami-ku,**
**Tokyo 167-0032 (JP)**

(72) Inventors:
• **NAGANO, Tetsuo**
**Suginami-ku, Tokyo 167-0032 (JP)**
• **KIKUCHI, Kazuya**
**Sakae-ku, Yokohama-shi, Kanagawa 247-000 (JP)**
• **KORESAWA, Mitsunori**
**Bunkyo-ku, Tokyo 112-0012 (JP)**
• **KOJIMA, Hirotatsu**
**Toshima-ku, Tokyo 171-0031 (JP)**

(74) Representative: **Polypatent**
**Postfach 11 07**
**51482 Overath (DE)**

(56) References cited:

WO-A-00/00819    CA-A- 2 295 880
JP-A- 5 180 773    JP-A- 9 101 262
JP-A- 10 088 124    JP-A- 2000 111 480
US-A- 5 656 433

• BAMBOT S B ET AL: "Potential applications of lifetime-based, phase-modulation fluorimetry in bioprocess and clinical monitoring" TRENDS IN BIOTECHNOLOGY, ELSEVIER, AMSTERDAM, NL, vol. 13, no. 3, March 1995 (1995-03), pages 106-115, XP004207135 ISSN: 0167-7799
• SIPIOR J ET AL: "Lifetime-based optical sensing of pH using resonance energy transfer in sol-gel films" SENSORS AND ACTUATORS B, ELSEVIER SEQUOIA S.A., LAUSANNE, CH, vol. 22, no. 3, December 1994 (1994-12), pages 181-188, XP004011062 ISSN: 0925-4005
• SELVIN P R ET AL: "LUMINESCENCE ENERGY TRANSFER USING A TERBIUM CHELATE: IMPROVEMENTS ON FLUORESCENCE ENERGY TRANSFER" PROCEEDINGS OF THE NATIONAL ACADEMY OF SCIENCES OF USA, NATIONAL ACADEMY OF SCIENCE, WASHINGTON, DC, US, vol. 91, October 1994 (1994-10), pages 10024-10028, XP001018013 ISSN: 0027-8424
• YUAN J ET AL: "FUNCTIONALIZATION OF FLUORESCENT LANTHANIDE COMPLEXES AND THEIR APPLICATIONS TO BIOTECHNOLOGY" BUNSEKI KAGAKU - JAPAN ANALYST, NIPPON BUNSEKI KAGAKKAI, TOKYO,, JP, vol. 48, no. 12, 1999, pages 1077-1083, XP002932633 ISSN: 0525-1931

**Description**

Technical Field

The present invention relates to a method of measuring a target substance in a solution by detecting fluorescence. More specifically, the present invention relates to a method of measuring a target substance in a solution by detecting fluorescence, by which the target substance is accurately measured with high sensitivity without using probes as a means to obtain specificity, such as proteins or nucleic acids labeled with fluorescent substances, and without being affected by background fluorescence.

Background Art

A method of measuring a target substance in a sample by detecting fluorescence (fluorescence method) enables convenient and highly sensitive measurement. The method can also be automated using an analyzer, such as an immuno-plate reader. Therefore, the method has been used in various fields including diagnostic test. In particular, the fluorescence method is used for assay measurements in high-throughput screening (HTS) by which lead compounds as drug candidates are chosen from thousands and tens of thousands of samples on the basis of various properties of fluorescence (fluorescence intensity, anisotropy, excitation energy transfer, fluorescence life and the like). The fluorescence method is highly suitable for application to HTS from viewpoints of high efficiency, convenience and the like, and the method is believed to become a major method for assay measurement in HTS in the future (Rogers, M.V., Drug Discovery Today, Vol. 2, pp.156-160, 1997).

In the method of measuring a target substance in a sample by detecting fluorescence, so-called background fluorescence, which is not derived from a target substance, may sometimes be produced. The background fluorescence is generated, for example, from endogenous substances other than a target substance in a sample which have auto-fluorescence; from fluorescent dye attached non-specifically to proteins or the like in a sample; or from a container (such as a plate) into which a target substance is filled. Background fluorescence is a common problem of the methods for measuring a target substance in a sample by detecting fluorescence, because any of the above background fluorescence affects sensitivity and specificity. Accordingly, a method for measurement which is free from the influence of background fluorescence has been required.

To avoid the influence of background fluorescence, a method using Time Resolved Fluorescence (TRF) measurement has been studied. The method with TRF uses the fact that measurement can be performed without disturbance of background fluorescence by irradiating with a pulsed excitation light, then providing delay time, followed by measuring long-lived fluorescence derived from lanthanoide ion complexes after contaminating-background fluorescence has been quenched, wherein the fact is based on the fluorescence life of a lanthanoide ion complex is so long as tens of micro seconds to several milliseconds, whilst the life of fluorescence that is produced from normal organic compounds and a cause of background fluorescence is as short as several nanoseconds to tens of nanoseconds.

However, a lanthanoide ion complex itself does not have any specificity to a target substance, and to obtain specificity, utilizations of antigen-antibody reaction (for example, use of lanthanoid ion complex-labeled specific antibodies for a target substance) or interaction between nucleic acid bases (for example, labeling of a single stranded DNA fragment capable of hybridizing to a target substance with a lanthanoid ion complex) and the like are essential. Accordingly, the lanthanoid ion complex cannot be applied for measurement of physiologically active species or vital reactions for which the above reaction or action is hardly applicable.

Fluorescence resonance energy transfer (FRET) is a phenomenon which is observed when the two types of fluorescent molecules, the donor and the acceptor, are present in a measurement system, fluorescence is observed in the acceptor even upon excitation of the donor. This phenomenon occurs because of the presence of overlap of fluorescence spectrum of the donor and absorbance (excitation) spectrum of the acceptor.

FRET is a method with high specificity whose detection principle is based on a change in relative distance or in relative configuration between a donor and an acceptor, and used in, for example, (1) measurement of intermolecular interaction which comprising the steps of labeling a protein and a ligand which specifically bind to each other with a donor and an acceptor, respectively, and detecting FRET generated upon binding; (2) measurement of a change in relative configuration of two particular positions by labeling each of two different particular positions in a single molecule with a donor and an acceptor, and detecting changes in FRET efficiency generated in response to any kind of stimulation; (3) measurement of an enzymatic activity comprising the steps of labeling both ends of a peptide sequence that can be specifically recognized by a target enzyme or a substrate analog containing a specific linkage (such as ester linkage) with a donor and an acceptor, and detecting changes in FRET efficiency before and after cleavage. However, there are problems in that preparations of reagents or the like before the measurements are inconvenient and highly costly, because a protein, a nucleic acid or the like is required to be labeled with a donor or an acceptor so as to provide a particular relative distance or a relative configuration of the donor and acceptor upon measurement.

In recent years, several methods combining TRF and FRET within a single measurement system have been reported as homogeneous time resolved fluorometry (HTRF). HTRF is reported to drastically improve detection limit in immunoassay, detection of intermolecular interaction and the like by using an europium ion complex as a donor and APC (fluorescent protein with its molecular weight of 104,000) or Cy5 (Cyanin-dye) as an acceptor. The result in this method is believed to be attributable to the use of TRF, which reduces background fluorescence so as to remarkably improve a S/N ratio. However, procedures to label proteins or the like with a donor or an acceptor is essential so as to provide a particular relative distance or a relative configuration between a donor and a acceptor upon measurement.

WO 00/00819 describes an assay method for measuring the pH-value in a probe by using FRET. The assay comprises a fluorescent metal ligand complex as donor and a pH indicator as acceptor. Ligand and acceptor are fixed in a matrix.

Selvin P, Hearst J., Proc Natl. Acad. Sci, vol. 91, p.10024-10028, 1994 describe terbium compounds as donor and tetramethylrhodamine as acceptor. The compounds are bound to an DNA Oligomer to measure FRET dependent on the structure of the DNA oligomer.

Disclosure of the Invention

An object of the present invention is to provide a method of measuring a target substance in a solution by detecting fluorescence, which is convenient and highly sensitive and capable of measuring a target substance without being affected by background fluorescence without using, as a means to obtain specificity, a probe which is a protein, a nucleic acid or the like labeled with a fluorescent substance.

The inventors of the present invention conducted various studies to achieve the foregoing object. As a result, they surprisingly found that no FRET is caused when a donor which, per se, has long-lived fluorescence and is capable of causing FRET and an acceptor for said donor are connected in extreme proximity, whilst that FRET is caused when the donor and the acceptor are not connected and freely movable.

The inventors conducted further studies and found that a target substance in a solution can be measured with extremely high sensitivity by allowing co-existence of a specific fluorescent probe by specifically reacting with the target substance in the sample, and a donor which, per se, has long-lived fluorescence of $10^{-5}$ to $10^{-3}$s and is capable of inducing FRET on the acceptor; the step of exciting the donor into a long-lived excitation state and to induce the fluorescence resonance energy transfer on the acceptor, wherein the fluorescence energy transfer is caused when the donor and acceptor are not connected and freely movable; and measuring the long-lived excitation fluorescence by the TRF. In addition, the inventors also found that the above method enabled measurement without influence of background fluorescence generated from auto-fluorescence derived from endogenous substances other than a target substance in a sample or from a container (for example, a plate) containing an injected target substance, and thereby enabled extremely accurate measurement. The present invention was achieved on the basis of these findings.

The present invention, thus provides a method of measurement of a target substance in a sample by means of fluorescence, which comprises:

the step of carrying out the measurement in the presence of:

(1) a specific probe which specifically reacts with the target substance forming an acceptor to generate fluorescence, and
(2) a donor which, per se, has long-lived fluorescence of $10^{-5}$ to $10^{-3}$s and is capable of inducing fluorescence resonance energy transfer to the acceptor, provided that the donor forms no binding to the specific acceptor by means of a chemical bond; and

the step of exciting the donor into a long-lived excitation state and to induce the fluorescence resonance energy transfer on the acceptor, wherein the fluorescence energy transfer is caused when the donor and acceptor are freely movable. According to a preferred embodiment of the aforementioned method, a method is provided which comprises the step of measuring the long-lived excitation fluorescence by time-resolved fluorescence measurement.

According to further preferred embodiments of the present invention, provided are the aforementioned method wherein the above donor is a lanthanoid ion complex; the aforementioned method wherein the above lanthanoid ion complex is an europium ion complex or a terbium ion complex; the aforementioned method wherein the above acceptor has a xanthene skeleton; the aforementioned method wherein the above donor is a terbium ion complex and the above acceptor has a rhodamine skeleton; and the above aforementioned wherein the target substance is nitrogen monoxide or a caspase.

Brief Explanation of Drawings

Figure 1 shows that in a system wherein $Tb^{3+}$ complex and DAR-M co-exist and in a system wherein $Tb^{3+}$ complex and DAR-MT co-exist, fluorescence resonance energy transfer (FRET) is induced from $Tb^{3+}$ complex toward DAR-M or DAR-MT; and in the presence of DAR-MT, long-lived fluorescence derived from DAR-MT is produced by FRET. In the figure, (A) shows the result of the system wherein $Tb^{3+}$ complex and DAR-M co-exist, and (B) shows the result of the system wherein $Tb^{3+}$ complex and DAR-MT co-exist.

Figure 2 shows the result of measurement of nitrogen monoxide by using DAR-M as a specific fluorescent probe by the method of the present invention.

Figure 3 shows a mode of overlap of the fluorescence spectrum of $Eu^{3+}$ complex and the absorption spectrum of SNR3.

Best Mode for Carrying out the Invention

The method of the present invention is for measurement of a target substance in a sample with fluorescence, and characterized to carry out the measurement in the presence of:

(1) a specific probe which specifically reacts with the target substance forming an acceptor to generate fluorescence, and

(2) a donor which, per se, has long-lived fluorescence of $10^{-5}$ to $10^{-3}$s and is capable of inducing fluorescence resonance energy transfer to the acceptor, the step of exciting the donor into a long-lived excitation state and to induce the fluorescence resonance energy transfer on the acceptor, wherein the fluorescence energy transfer is caused when the donor and acceptor are not connected and freely movable and preferably, to measure the long-lived excitation fluorescence of the acceptor resulting from the above FRET by using time resolved fluorescence measurement (TRF). It is required that the donor and the specific fluorescent probe form no binding by means of a chemical bond, and each substance exists independently in the measurement system.

Examples of types of the target substances are not particularly limited. Examples include in vivo molecules, such as nitrogen monoxide, $Ca^{2+}$ and $Zn^{2+}$, and hydrolases such as a caspase. Types of the specific fluorescent probes that react with the target substance to generate fluorescence are not particularly limited. Any probes can be used so long as they can specifically react with the target substance, and can generate fluorescence as a result of the reaction. The term "specifically react" in the specification normally means a property of reacting substantially only with a target substance, without substantially reacting with other components contained in the sample. However, a probe can also be used which has a lowest limit of specific reactivity that enables measurement of a target substance, and accordingly, the term should be by no means construed in any limiting sense. Types of the samples containing the target substance are not particularly limited, and any samples can be used. For example, the samples encompass natural samples such as biological samples as well as artificial samples.

Examples of the biological samples include those isolated ex vivo, for example, blood (serum and blood plasma), body fluids such as urine, tissues, or cells. Examples of the artificial samples include, but are not limited thereto, tissues and cells derived from animals or plants produced by gene recombination, as well as cells or the like containing non-natural type proteins produced by gene recombination. The term "measurement" in the present specification should be construed in the broadest sense including measurements with variety of purposes such as detection, quantification, qualification and the like.

Mechanisms of generation of fluorescence as a result of reaction of a specific fluorescent probe with a target substance are also not particularly limited. Examples include, but are not limited thereto, where a specific fluorescent probe per se has a substantially non-fluorescent chemical structure before reaction with a target substance, whilst it changes structure so as to have fluorescence by the reaction with the target substance; and where a fluorescent substance is connected in a molecule of a specific fluorescent probe in a manner to cause quenching, and the linkage is cleaved upon reaction with a target substance.

Examples where a specific fluorescent probe per se has a substantially non-fluorescent chemical structure before reaction with a target substance, whilst it changes structure so as to have fluorescence by the reaction with the target substance include a diaminofluorescein derivative generating fluorescence by reaction with nitrogen monoxide (Japanese Patent Laying-Open Publication (Kokai) No. (Hei) 10-226688) and a diaminorhodamine derivative (International Publication WO99/01447), fluorescent probe for Zinc (the specification of Japanese Patent Application No. (Hei) 11-040325), and a probe for measuring single oxygen (International Publication WO99/51586). For example, the diaminorhodamine derivative described in International Publication WO99/01447 specifically reacts with nitrogen monoxide to change its structure so as to have a triazole ring and emit fluorescence on the basis of the structural change.

An example where a fluorescent substance is connected in a molecule of a specific fluorescent probe in a manner to cause quenching, and the linkage is cleaved upon reaction with a target substance includes PhiPhiLux-G2D2 generating fluorescence by reaction with a caspase (OncoImmuni) (New Apoptosis Experimental Protocol, 2nd ed, YODOSHA, pp201-204, 1999). PhiPhiLux-G2D2 has a structure in which each of the ends of a specific amino acid sequence (GDEVDGID), that is cleaved with caspase-3, -7 and the like, is bound with one molecule of rhodamine. Since rhodamines at both ends form a dimeric structure, the molecule exists in a state wherein fluorescence is quenched. When the linkage of two molecules of rhodamine is cleaved between GDEVD and GID by reaction with a caspase, the quenching effect is lost and fluorescence is generated.

Examples of a donor which, per se, has long-lived fluorescence and is capable of inducing fluorescence resonance energy transfer to a specific fluorescent probe as an acceptor include lanthanoid ion complexes in which a ligand forms a chelate with a lanthanoid ion, such as $Eu^{3+}$ (europium ion), $Tb^{3+}$ (terbium ion), $Sm^{3+}$ (samarium ion) or $Dy^{3+}$ (dysprosium ion). The lanthanoid ion complex can be obtained by ordinary methods. For example, DTPA-cs124, which is an analogue of DTPA (Diethylenetriaminepentaacetic Acid) known as a ligand that forms a chelate with a lanthanoid ion, is synthesized by a method of Selvin et al (Selvin P.R. et al, J. Am. Chem. Soc., Vol.117, pp.8132-8138, 1995), and the resulting ligand is mixed with a lanthanoid ion in a solution to obtain a lanthanoid ion complex.

An optimum combination of a specific fluorescent probe and the above donor (for example, a lanthanoid ion complex) can be chosen so as to induce optimal fluorescence resonance energy transfer to the specific fluorescent probe as an acceptor, and to have the acceptor generate long-lived excitation fluorescence measurable by time resolved fluorescence measurement. As for fluorescence resonance energy transfer, explanations are given in Stryer L., Ann. Rev. Biochem., Vol. 47, pp.819-846, 1978 or the like. Whether or not fluorescence resonance energy transfer is induced can be accurately determined according to the method described in the above literature. For example, the judgment can be appropriately conducted experimentally by referring to the measurement methods specifically described in the examples of the specification.

Long-lived fluorescence generally means fluorescence having a lifetime ranging from about $10^{-5}$ sec to $10^{-3}$ sec. The fluorescence lifetime of a donor is generally about $10^{-3}$ sec, and that of long-lived excitation fluorescence resulting from fluorescence resonance energy transfer is generally about $10^{-4}$ sec. Further, time resolved fluorescence measurement is described in detail in Hammila I. & Webb S., Drug Discovery Today, Vol.2, pp. 373-381, 1997, and the specific techniques are shown in the examples of the present specification. Accordingly, one of ordinary skilled in the art can easily perform the measurement method.

Examples

The present invention will be more specifically explained with reference to the following examples. However, the scope of the present invention is not limited to the following examples.

Example 1: Preparation of specific fluorescent probe (DAR-M)

DAR-1 [3,6-bis(diethylamino)-9-(3,4-diamino-2-carboxyphenyl)xanthylium, intramolecular salt] prepared by the method described in International Publication WO99/01447 was dissolved in ethanol. The mixture was added with methyl iodide at 1.7 equivalences to DAR-1, and then the temperature was raised to 80°C. A rate of consumption of the raw material and a rate of production of the dimethyl product were monitored with TLC every hour, and 1.7 equivalences of methyl iodide was further added, and when a desired product was produced, the reaction was terminated. The product was purified by silica gel chromatography and preparative TLC to obtain DAR-M[3,6-bis(diethylamino)-9-[3-amino-4(N-methylamino)-2-carboxyphenyl]xanthyli um, inner salt].
m.p. 150-154°C
$^{1}$H–NMR (300 MHz, $CDCl_3$) δ 1.13 (12H, t, J=7.0), 2.86 (3H, s), 3.33 (8H, q, J=7.0), 6.37-6.43 (5H, m), 6.75 (1H, d, J=7.9), 6.81 (2H, d, J=9.0)
FAB-MS 487 (M$^{+}$+1)

DAR-M obtained in the above example was dissolved in methanol, and the solution was bubbled with nitrogen monoxide gas and then the solvent was evaporated. The product was purified with preparative TLC to obtain DAR-MT [3,6-bis(diethylamino)-9-[4-carboxy-1-methylbenzotriazole-5-il]xanthylium, inner salt].
m.p. 155-160°C
$^{1}$H–NMR (300 MHz, $CDCl_3$) δ 1.12 (12H, t, J=7.1), 3.32 (8H, q, J=7.1), 4.37 (3H, s), 6.31 (2H, dd, J=9.0, 2.5), 6.43 (2H, d, J=2.5), 6.58 (2H, d, J=9.0), 7.26 (1H, d, J=8.6), 7.83 (1H, d, J=8.6)
FAB-MS 498 (M$^{+}$+1)

Example 2

(1) Preparation of sample

DTPA-cs124, which is an analogue of DTPA (Diethylenetriaminepentaacetic Acid), was synthesized by the method of Selvin et al (Selvin P.R. et al, J. Am. Chem. Soc., Vol.117, pp.8132-8138, 1995). A DMSO solution of 10mM DTPA-cs124 and an equivalent amount of 10mM $TbCl_3$ aqueous solution were mixed, and then diluted with 0.1M Tris-HCl buffer (pH 8.8) so as to become a final concentration of 10 $\mu$M. The mixture was then allowed to stand for 30 min or more to form terbium ion complex ($Tb^{3+}$ complex). The solutions of the above $Tb^{3+}$ complex were added with DAR-M or DAR-MT obtained in Example 1 at final concentrations of 1.0, 2.0, 3.0, 5.0 and 10.0 $\mu$ M, and then the mixtures were subjected to measurements. Samples each solely containing DAR-M or DAR-MT without coexistence with $Tb^{3+}$ complex were prepared, and then used for measurement as controls.

(2) Measurement of TRF spectrum

The spectra of the samples obtained in (1) above were measured under the following photometer setting conditions.
Mode: Phosphate, Excitation: 328nm, Delay Time: 50$\mu$s, Flash Count: 1, Gate Time: 1.00 ms, Cycle Time: 20 ms, Slit width: 2.5 nm (common for Excitation and Emission), Scan speed: 900 nm/min

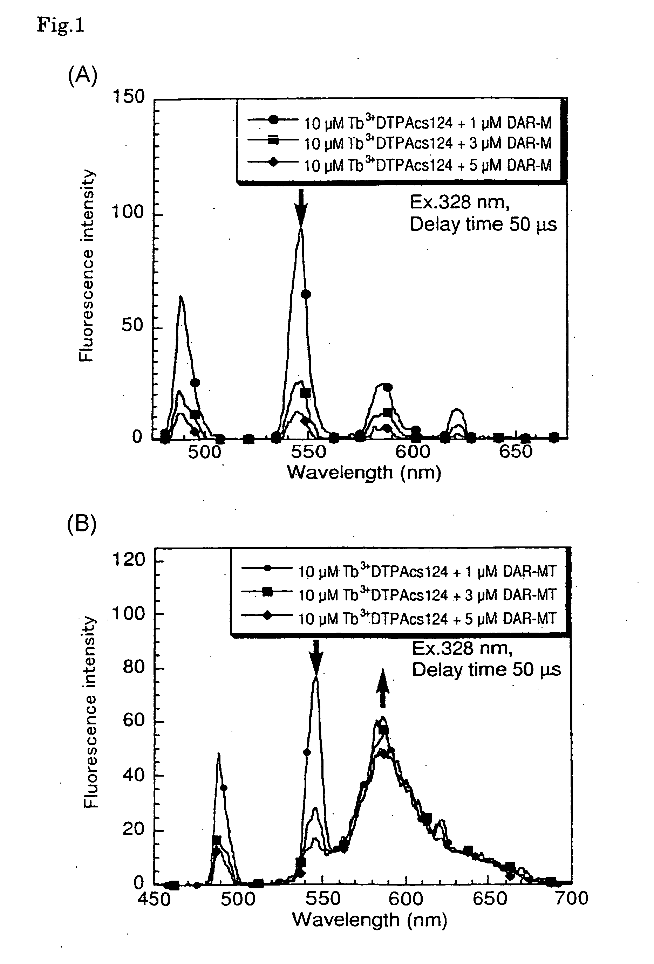

The results are shown in Figure 1. In the system wherein $Tb^{3+}$ complex and DAR-M coexisted (Fig. 1A), a decrease in fluorescence intensity at 545 nm derived from $Tb^{3+}$ chelate was observed in a DAR-M concentration-dependent manner. In contrast, in the system wherein $Tb^{3+}$ complex and DAR-MT coexisted (Fig. 1B), a decrease in fluorescence intensity was observed at 545 nm derived from $Tb^{3+}$ complex in a DAR-MT concentration-dependent manner, and fluorescence having a peak point at 584 nm appeared which was distinguishable from the fluorescence derived from $Tb^{3+}$ chelate. In the system where DAR-M or DAR-MT did not coexist with $Tb^{3+}$ complex, no fluorescence was observed when spectra were measured under the same conditions. It was concluded that DAR-M and DAR-MT have no long-lived fluorescence greater than 50 $\mu$s of Delay Time. These results revealed that in the system wherein $Tb^{3+}$ complex and DAR-M coexisted, FRET occurred from $Tb^{3+}$ complex to DAR-M, but only a decrease in fluorescence intensity of $Tb^{3+}$ chelate was observed because DAR-M has no fluorescence. Whilst in the system wherein $Tb^{3+}$ complex and DAR-MT coexisted, FRET occurred from $Tb^{3+}$ complex to DAR-MT, and the long-lived fluorescence of DAR-MT was produced by the FRET.

Measurement of fluorescence lifetime

Fluorescence intensity was measured every 50 $\mu$s for samples of (1) above under the following photometer setting conditions, and then the data obtained were fitted to the formula, $I = Ioexp(-t/\tau)$ to obtain the decay curves of fluorescence. With the resulting decay curves, fluorescence lifetime ($\tau$) was obtained.
Mode: Short Phos. Decay, Excitation: 328 nm, Emission: 545 nm or 584 nm, Flash Count: 1, Gate Time: 0.01 ms, Cycle Time: 20 ms, Slit width: 10 nm, Integ. Time: 1.0 s

The results are shown in Table 1.

In the system wherein $Tb^{3+}$ complex and DAR-M coexisted (the column of DAR-M in Table 1), fluorescence lifetime at 545 nm derived from $Tb^{3+}$ complex was shortened in a DAR-M concentration-dependent manner. In contrast, in the system wherein $Tb^{3+}$ complex and DAR-MT coexisted (the column of DAR-MT in Table 1), in a DAR-MT concentration-dependent manner, fluorescence lifetime at 545 nm derived from $Tb^{3+}$ complex and the lifetime of another fluorescence that appeared at 584 nm separately from fluorescence derived from $Tb^{3+}$ complex were both shortened while coinciding with each other at an order of $\mu$s. As described above, similar to the results of (2) above, the newly appeared fluorescence at 584 nm separately from the fluorescence derived from $Tb^{3+}$ chelate was shown to be long-lived fluorescence of DAR-MT resulting from FRET that occurred from $Tb^{3+}$ complex to DAR-MT.

Table 1

| Run | Sample | DAR-M $\tau_{545}$(ms) | DAR-MT $\tau_{545}$(ms) | DAR-MT $\tau_{584}$(ms) |
|-----|--------|------------------------|-------------------------|-------------------------|
| 1 | 10 $\mu$ M $Tb^{3+}$DTPAcs124 | 1.52 | 1.52 | - |
| 2 | Run1+1 $\mu$ M DAR derivative | 0.59 | 0.45 | 0.45 |
| 3 | Run1+2 $\mu$ M DAR derivative | 0.29 | 0.29 | 0.29 |

(continued)

| Run | Sample | DAR-M | DAR-MT | |
|---|---|---|---|---|
| | | $\tau_{545}$(ms) | $\tau_{545}$(ms) | $\tau_{584}$(ms) |
| 4 | Run1+3 $\mu$M DAR derivative | 0.22 | 0.19 | 0.19 |
| 5 | Run1+5 $\mu$M DAR derivative | 0.13 | 0.13 | 0.13 |
| 6 | Run1+10 $\mu$M DAR derivative | 0.08 | 0.08 | 0.08 |

Example 3: Measurement of nitrogen monoxide

Preparation of reagent for measuring nitrogen monoxide

A DMSO solution of 10 mM DTPA-cs124 and an equivalent amount of 10 mM TbCl$_3$ aqueous solution were mixed, and then diluted with 0.1 M sodium phosphate buffer (pH 7.0) so as to become a final concentration of 10 $\mu$M. Then the mixture was allowed to stand for 30 min or more to obtain Tb$^{3+}$ complex. Further, the Tb$^{3+}$ complex was added with 10 mM DAR-M DMSO solution at a final concentration of 3.0$\mu$M to prepare a reagent for measuring nitrogen monoxide.

(2) Measurement method

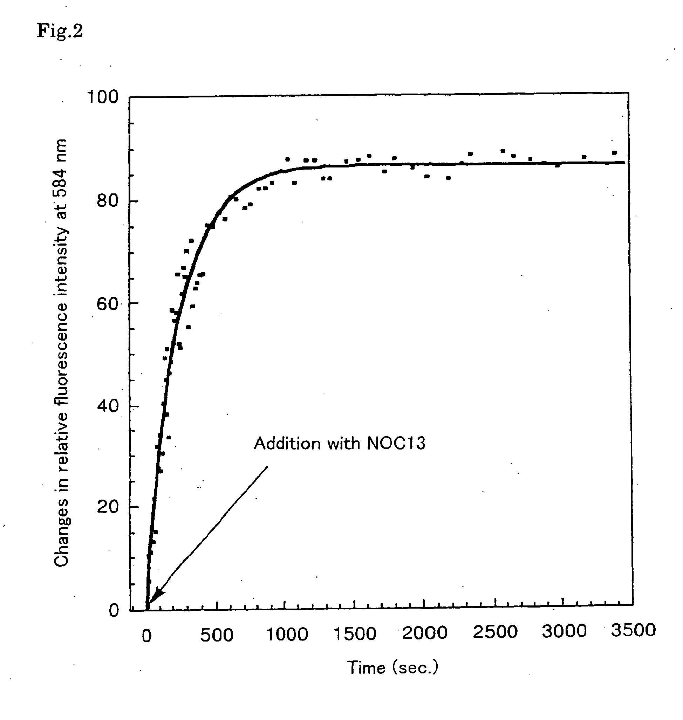

The reagent for measuring nitrogen monoxide obtained in (1) above was filled in a fluorescent cell, and then changes in fluorescence intensity at 328 nm excitation wavelength and at 584 nm fluorescence wavelength were measured with time while the solution was stirring. At 120 sec after the start of the measurement, NOC13 was added at a final concentration of 10 $\mu$M as a donor of nitrogen monoxide gradually releasing nitrogen monoxide in the buffer. Measurement was continued up to 3600 sec. A solution of Tb$^{3+}$ complex or DAR-M at a concentration equal to that of the reagent for measuring nitrogen monoxide was prepared as a control.
Apparatus: Hitachi F-4500,
Mode: Measurement with changes in fluorescence time, Excitation: 328 nm, Emission: 584 nm, Slit width: 2.5 nm (common for Excitation and Emission), Photomultiplier voltage: 950 V
Figure 2 shows changes with time in net fluorescence intensity derived from the detection of nitrogen monoxide obtained by subtracting changes with time in fluorescence intensity of DAR-M as a sole reagent from that of the reagent for measuring nitrogen monoxide. Immediately after the addition with NOC13, increases with time were observed in fluorescence intensity at 584 nm.

Example 4 (Reference example)

A substance (SNR3), which efficiently causes fluorescence resonance energy transfer, was used as a specific fluorescent probe model, and by a combination with a donor, fluorescence resonance energy transfer was induced and then long-lived excitation fluorescence generated from the substance was measured.

(1) Preparation of SNR3

(a) 2-hydroxy-4-tetrahydroquinolizino[1,9-hi](2'-carboxybenzoyl)benzene

Phthalic anhydride (2 g, 13.5 mmol) and 8-hydroxyquinolizine (1 g, 5.28 mmol) were mixed in toluene (30 ml), and the refluxed with heating overnight. The solvent was evaporated, and the residue was purified by silica gel column chromatography (AcOEt / CH$_2$Cl$_2$ = 1/1) to obtain a target compound (yield 40%).
$^1$H-NMR(300 MHz, DMSO-d$_6$) $\delta$ 1.68-1.91 (m, 4H), 2.32-2.46 (t, 2H,J=6.2 Hz), 2.55-2.63 (t, 2H, J=6.2 Hz), 3.15-3.30 (m, 4H), 6.40 (s, 1H), 7.32 (d, 1H, J=6.6 Hz), 7.55-7.68 (m, 2H), 7.94 (d, 1H, J=7.5 Hz), 12.95 (s, br 1H)

(b) 6-N,N-diethylamino-1-naphthol

6-amino-1-naphthol (2 g, 12.6 mmol), iodoethane (10 g, 64 mmol) and triethylamine (1 ml) were mixed in anhydrous dimethyl formamide (5 ml), and then the mixture was stirred at 120°C for 5 hours. The reaction mixture was added with 200 ml of dichloromethane, washed with water, and then washed with saturated saline. The organic layer was dried, and then the solvent was evaporated to obtain a residue. The residue was purified by silica gel column

chromatography (n-hexane : $CH_2Cl_2$=1 : 9) to obtain a target compound (yield 32%).
$^1$H-NMR(300 MHz, DMSO-$d_6$) δ 1.20 (t, 6H,J=7.0 Hz), 3.44 (q, 4H), 6.47 (dd, 1H, J=7.0, 1.3 Hz), 6.84 (d, 1H, J=2.6 Hz), 7.07 (dd, 1H, J=9.3, 2.6 Hz), 7.21 (m, 2H), 8.00 (d, 1H, J=9.4 Hz)

(c) 3-diethylamino-10-tetrahydroquinolizino [1,9-hi]-9-[2'-carboxyphenyl]-benzo [c] xanthylium (SNR3)

2-hydroxy-4-tetrahydroquinolizino[1,9-hi](2'-carboxybenzoyl)benzene obtained in (a) above (60 mg, 0.18 mmol) and 6-N,N-diethylamino-1-naphthol (40 mg, 0.18 mmol) obtained in (b) above were dissolved in methanesulfonic acid (2 ml), and then the mixture was stirred at 85°C for 12 hours. The reaction mixture was cooled, and then poured into about 500 ml of ice-water. The mixed solution was neutralized with 2N NaOH aqueous solution while being cooled with ice-water, and then 5 ml of concentrated hydrochloric acid was added to make the solution acidic. The resulting precipitate was collected by filtration, and then purified by silica gel column chromatography (10% MeOH /$CH_2Cl_2$) to obtain a target compound (yield 20%).
$^1$H-NMR(300 MHz, DMSO-$d_6$) δ 1.23 (t, 6H,J=7.0 Hz, a); 2.12 (m, 8H, b); 3.11 (m, 4H, c); 4.13 (q, 4H, J=7.9 Hz, d); 6.24 (s, 1H, e); 6.59 (d, 1H, J=8.8 Hz, f); 6.79 (d, 1H, J=2.6 Hz, g); 7.17 (m, 3H, h, i, j); 7.62 (m, 2H, k,l); 8.04 (m, 1H, m); 8.35 (d, 1H, J=9.2 Hz, n) FAB-MS : 517 (M$^+$)

(2) Measurement of spectrum

Ultraviolet and visible absorption spectra were measured using Shimadzu UV-1600 (sampling pitch : 0.2 nm or 0.5 nm, and a low speed was used from among 6 stages of scanning speed). Fluorescence spectra were measured using Perkin Elmer LS50B (scan speed : 900 nm/min, and slit width at both excitation side and fluorescence side : 2.5 nm). The spectrum overlap integral J of the fluorescence spectrum of Eu$^{3+}$ chelate and the absorption spectrum of SNR3 was calculated using the following formula.

$$J = \frac{\int \varepsilon_A(\lambda) \, F_D(\lambda) \, \lambda^4 d\lambda}{\int F_D(\lambda) \, d\lambda}$$

$$( \text{cm}^{-1}\text{nm}^4\text{M}^{-1})$$

$\varepsilon A(\lambda)$: molar absorption coefficient of acceptor
$F_D(\lambda)$: fluorescence intensity of donor
$\lambda$: wavelength

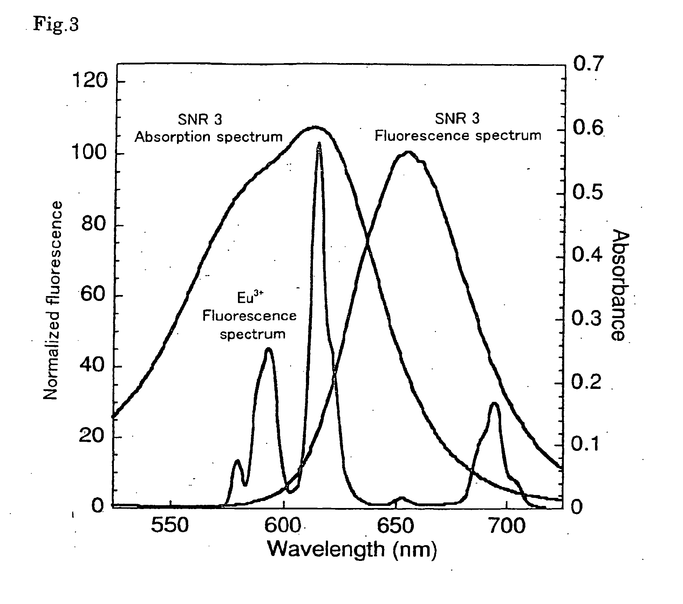

A DMSO solution of SNR3 (10 mM) was diluted with potassium phosphate buffer (pH 7.4) to prepare 10 μM solution, and then the solution was used for spectral measurement. Ultraviolet and visible absorption spectra and fluorescence spectra were measured, and then the spectral overlap integral J of the fluorescence spectrum of Eu$^{3+}$ complex and the absorption spectrum of SNR3 was calculated. The results are shown in Figure 3. The maximum fluorescence intensity of SNR3 and that of Eu3$^+$ chelate were both taken as 100, and were shown as being overlapped with the absorption spectrum of 10 μM SNR3 solution (0.1 M potassium phosphate buffer, pH7.4). SNR3 showed fluorescence properties of $\lambda$ ex = 615 nm and $\lambda$ em = 655 nm, and the spectral overlap integral with the fluorescence spectrum of Eu$^{3+}$ complex was 7.34 $\times 10^{15}$ (nm$^4$cm$^{-1}$M$^{-1}$). This value is large enough to induce FRET, as compared to the degree of the overlap integral (6.55$\times 10^{15}$ nm$^4$cm$^{-1}$M$^{-1}$, Selvin, P.R., et al., J. Am. Chem. Soc., 116, 6029-6030, 1994) of the absorption spectrum of the long wavelength excitation fluorescent substance Cy5 and the fluorescence of Eu$^{3+}$ chelate.

Industrial Applicability

According to the present invention, a target substance contained in a biological sample or the like can be accurately measured with high sensitivity without being influenced by background fluorescence. Further, the method does not require, as a means to obtain specificity, the use of probes that are proteins, nucleic acids or the like labeled with fluorescent substances. Accordingly, the method achieves easy preparation of reagents, and the method can be applied to measurement of biologically active species and biological reactions for which antigen-antibody reaction, interaction of nucleic acid bases and the like cannot be utilized.

**Claims**

1. A method of measurement of a target substance in solution by means of fluorescence, which comprises:

   the step of carrying out the measurement in the presence of:

   (1) a specific probe which specifically reacts with the target substance forming an acceptor to generate fluorescence, and
   (2) a donor which, per se, has long-lived fluorescence of $10^{-5}$ to $10^{-3}$ seconds and is capable of inducing fluorescence resonance energy transfer to the acceptor, provided that the donor forms no binding to the specific acceptor by means of a chemical bond; and the step of exciting the donor into a long-lived excitation state and to induce the fluorescence resonance energy transfer on the acceptor, wherein the fluorescence energy transfer is caused when the donor and the acceptor are not connected and freely movable.

2. The method according to claim 1, which comprises the step of measuring the long-lived excitation fluorescence by time-resolved fluorescence measurement.

3. The method according to claim 1 or claim 2, wherein the donor is a lanthanoid ion complex.

4. The method according to claim 3, wherein the lanthanoid ion complex is an europium ion complex or a terbium ion complex.

5. The method according to any one of claims 1 to 4, wherein the acceptor has a xanthene skeleton.

6. The method according to claim 4, wherein the donor is a terbium ion complex and the acceptor has a rhodamine skeleton.

7. The method according to any one of claims 1 to 6, wherein target substance is nitrogen monoxide or a caspase.

**Patentansprüche**

1. Verfahren zur Messung einer Zielsubstanz in Lösung mittels Fluoreszenz, umfassend:

   den Schritt des Durchführens der Messung in Gegenwart:

   (1) einer spezifischen Sonde, die spezifisch mit der Zielsubstanz reagiert, um einen Akzeptor zu formen und eine Fluoreszenz zu erzeugen; und
   (2) eines Donors, der *per se* eine langlebige Fluoreszenz von $10^{-5}$ bis $10^{-3}$ Sekunden aufweist und in der Lage ist, einen Fluoreszenzresonanzenergie-Transfer zum Akzeptor herbeizuführen, mit der Maßgabe, dass der Donor nicht über eine chemische Bindung an den spezifischen Akzeptor bindet; und

   den Schritt des Anregens des Donors zu einem langlebigen Anregungszustand und des Herbeiführens des Fluoreszenzresonanzenergie-Transfers auf den Akzeptor, wobei der Fluoreszenzenergie-Transfer bewirkt wird, wenn Donor und Akzeptor nicht verbunden und frei beweglich sind.

2. Verfahren nach Anspruch 1, umfassend den Schritt des Messens der langlebigen Anregungsfluoreszenz durch zeitaufgelöste Fluoreszenzmessung,

3. Verfahren nach Anspruch 1 oder 2, wobei der Donor ein Lanthanidenionen-Komplex ist.

4. Verfahren nach Anspruch 3, wobei der Lanthanidenionen-Komplex ein Europiumionen-Komplex oder ein Terbiumionen-Komplex ist.

5. Verfahren nach einem der Ansprüche 1 bis 4, wobei der Akzeptor ein Xanthen-Gerüst aufweist.

6. Verfahren nach Anspruch 4, wobei der Donor ein Terbiumionen-Komplex ist und der Akzeptor ein Rhodamin-Gerüst aufweist.

**7.** Verfahren nach einem der Ansprüche 1 bis 6, wobei die Zielsubstanz Stickstoffmonoxid oder eine Caspase ist.

**Revendications**

**1.** Procédé de mesure d'une substance cible en solution par fluorescence, comprenant :

l'étape consistant à réaliser la mesure en présence de :

(1) une sonde spécifique qui réagit spécifiquement avec la substance cible formant un accepteur pour générer de la fluorescence, et
(2) un donneur, qui en soi possède une fluorescence de longue durée de vie allant de $10^{-5}$ à $10^{-3}$ secondes et est capable d'induire le transfert d'énergie de résonance par fluorescence à l'accepteur, à la condition que le donneur ne forme aucune liaison avec l'accepteur spécifique au moyen d'une liaison chimique ; et

l'étape consistant à exciter le donneur pour qu'il passe dans un état d'excitation de longue durée de vie et induise le transfert d'énergie de résonance par fluorescence sur l'accepteur, où le transfert d'énergie par fluorescence est provoqué quand le donneur et l'accepteur ne sont pas liés et sont librement mobiles.

**2.** Procédé selon la revendication 1, comprenant l'étape consistant à mesurer la fluorescence d'excitation de longue durée de vie par la mesure de la fluorescence à résolution temporelle.

**3.** Procédé selon la revendication 1 ou 2, dans lequel le donneur est un complexe d'ions de lanthanoïde.

**4.** Procédé selon la revendication 3, dans lequel le complexe d'ions de lanthanoïde est un complexe d'ions d'europium ou un complexe d'ions de terbium.

**5.** Procédé selon l'une quelconque des revendications 1 à 4, dans lequel l'accepteur possède un squelette de xanthène.

**6.** Procédé selon la revendication 4, dans lequel le donneur est un complexe d'ions de terbium et l'accepteur possède un squelette de rhodamine.

**7.** Procédé selon l'une quelconque des revendications 1 à 6, dans lequel la substance cible est un monoxyde d'azote ou une caspase.

Fig.1

(A)

(B)

Fig.2

Fig.3

**EP 1 271 133 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0000819 A
- JP 10226688 A
- WO 9901447 A
- JP 11040325 A
- WO 9951586 A

**Non-patent literature cited in the description**

- **ROGERS, M.V.** *Drug Discovery Today,* 1997, vol. 2, 156-160
- **SELVIN P ; HEARST J.** *Proc Natl. Acad. Sci,* 1994, vol. 91, 10024-10028
- New Apoptosis Experimental Protocol. YODOSHA, 1999, 201-204
- **SELVIN P.R. et al.** *J. Am. Chem. Soc.,* 1995, vol. 117, 8132-8138
- **STRYER L.** *Ann. Rev. Biochem.,* 1978, vol. 47, 819-846
- **HAMMILA I. ; WEBB S.** *Drug Discovery Today,* 1997, vol. 2, 373-381
- **SELVIN, P.R. et al.** *J. Am. Chem. Soc.,* 1994, vol. 116, 6029-6030